# EUROPEAN PATENT APPLICATION

(11) **EP 1 222 934 A2**
(43) Date of publication of application: **17.07.2002**
(21) Application number: 01310943.4
(22) Date of filing: 28.12.2001
(51) Int. Cl.: A61L 2/18

(54) **Bottle sterilizing system and method**

(30) Priority: 03.01.2001 US 753437
(71) Applicant: THE QUAKER OATS COMPANY, Chicago, Illinois 60610-4714 (US)
(72) Inventor: Raniwala, Subodh K., Mundelein, Illinois 60060 (US)
(74) Representative: Schlich, George William

(57) **Abstract**

A system and method for sterilizing bottles is provided. The system has structure for producing and introducing a source of liquid sterilizing agents in the form of discrete particles onto the bottle surface to form a thin liquid film thereon. Structure is provided for removing the sterilizing agent from the bottle surface after the desired degree of sterilization is achieved.

## Description

### FIELD OF THE INVENTION

The present invention relates to a system, method and apparatus for sterilizing bottles by introducing a sterilizing agent in the form of discrete atomized liquid droplets onto the bottle's surfaces.

### BACKGROUND OF THE INVENTION

In the high-speed automated bottling of beverages, it is desirable to fill the bottles in such a manner as to achieve a product shelf life as long as possible. In a cold-fill bottling process where the use of chemical additives or pasteurization is impractical, a germ-free bottle is necessary to minimize the chance that bacteria, spores or other microorganisms may contaminate the beverage during the filling stage, thereby reducing the shelf life of the bottled product.

In order to achieve this high degree of sterility, cold-fill systems typically incorporate the beverage bottling stage in a closed system, otherwise known as a clean room. A clean room is often used to minimize potential contamination and to substantially maintain a desired sterile environment level once it has been obtained.

Although the use of a clean room reduces the possibility of contamination, fill bottles, in particular, should be free from contamination as well. It is known in the art to apply sterilizing agents such as pressurized steam or liquid chemical solutions to sterilize bottles immediately prior to the beverage fill stage. These known methods, however, have certain drawbacks for a number of reasons. First, pressurized steam is not ideal to sanitize plastic (PET) bottles due to temperature deformation. Secondly, chemical spray or chemical bath approaches both require relatively large volumes of sterilizing solution to achieve proper sterilization. This is economically undesirable. Furthermore, the use of chemical sterilizing solutions requires excessive energy use and substantial amounts of equipment to maintain the solution at the required temperature to achieve the proper degree of sterilization.

A need therefore exists to apply sterilizing agent in a more uniform and energy efficient manner to all surfaces of a bottle while simultaneously ensuring that the required degree of sterilization is obtained.

### SUMMARY OF THE INVENTION

In accordance with one aspect of the present invention, a system, method and apparatus for sterilizing bottles in a uniform and efficient manner is provided that is particularly useful in a cold-fill bottling process. The present invention can reduce the amount of sterilizing agent required and can eliminate the need for post-bottling pasteurization.

In accordance with one aspect of the present invention, a system introduces a sterilizing agent in the form of discrete atomized liquid droplets onto the surface of a bottle to be sterilized. Once contacting the bottle surfaces, these droplets form a thin liquid film thereon. The film is maintained on the surface for a sufficient time and in sufficient concentration so that the sterilizing agent reduces the microorganism concentration on the surfaces to the desired level of sterility. When introducing the sterilizing agent, any bottle orientation may be used. Preferably, the bottle is inverted before the sterilizing agent is introduced. The inverted bottle is more readily adaptable to a sterilizing agent removal process, such as rinsing with water. Thus, the present action is particularly suited to sterilizing the interior of a bottle or other container as well as the exterior of such bottle or container. Thereafter, the bottle can be filled with a beverage as desired.

As used herein, "atomized" means a droplet size in the range of about 1 millimicron to 1000 microns (1 millimeter) in diameter. As such, the droplets may or may not be visible to the naked eye. The sterilizing agent may be atomized by any suitable method known to those skilled in the art. This may include but not be limited to techniques such as applying ultrasonic energy to liquid and forming a suspension of atomized particles, or utilizing a fog generator, an aspirator or a hydraulic atomizer nozzle. Given this size range, the atomized droplets may be introduced in various physical states including but not limited to a fog, a nebula, a vapor, a mist or an aerosol suspension.

Preferably, an air atomizer nozzle is used. The spray angle can vary from 0°, a straight-line jet, to a full-cone spray of 360°. The desired concentration of sterilizing agent is achieved by adjusting the sterilizing agent/air ratio of the nozzle pressure. Likewise, the flow rate and the droplet size can also be varied by adjusting the nozzle discharge aperture. By applying liquid sterilizing agent to bottles in the form of atomized liquid droplets, the normal volume usage of liquid sterilizing solution can be reduced by about 90% or 95% or more compared to traditional spray sterilizing methods in which a stream of liquid solution is sprayed into the bottles.

For purposes of the present invention, the term "surface(s)," refers to any bottle surface defined by the interior and exterior material layer of the bottle's shape. The bottle surface would thereby include any exposed convex or concave surfaces thereof.

After application of the sterilizing agent to the desired surface(s) of the bottle, the sterilizing agent is allowed to remain on the surface(s) for sufficient time to effect the desired degree of sterilization. Residual sterilizing agent is then removed from the bottle. This can be performed by rinsing the bottle with water, either by spraying the bottle with rinse water or immersing the bottle in a water bath. When spray rinsing the bottle, it is preferable to have the bottle inverted so that gravity assists in draining the rinse water from the bottle interior. After rinsing, if inverted, the bottle is turned up for filling.

In accordance with another aspect of the invention, the sterilizing agent is a liquid that is evaporable or washable without leaving any substantial residue. One such sterilizing agent is Oxonia™. Oxonia™ is an aqueous mixture of hydrogen peroxide, peracetic acid and inert ingredients. When such a sterilizing agent is used, the bottle may or may not need to be rinsed with water. Also, the sterilizing agent can be adequately removed by applying compressed air to the bottle until all the surfaces are sufficiently dry, which may physically remove the sterilizing agent from the surface as well as hasten evaporation.

In accordance with a preferred embodiment of the present invention, the atomized droplets contact the bottle by impinging the bottle surface. After impingement, the droplet collapses and the liquid sterilizing agent adheres to the bottle surface, generally covering more surface area than immediately after impingement. As more droplets impinge upon the surface, the collapsed droplets disperse or spread and wet the surface by creating a thin film of sterilizing agent. Impingement by a sufficient number of droplets per unit area of bottle surface (i.e., droplet density) creates a continuous thin film on the bottle surface.

In a preferred embodiment of the present invention, the atomized droplets are introduced in sufficient density so as to condense upon the bottle's surface and form a continuous thin film of sterilizing solution thereon. Condensation may be enhanced by supersaturating the sterilizing solution. Condensation may also be promoted by creating a temperature differential between the sterilizing solution and the bottle surface in which the bottle is colder than the surrounding atmosphere. Preferably, this occurs in a closed chamber or room. If a temperature differential is utilized, it can be on the order of 3°C or more as desired. The closed chamber helps maintain the temperature of the sterilizing agent when it is introduced at an elevated temperature. Moreover, the atmospheric pressure in the closed chamber may also be increased to promote condensation of the atomized droplets upon the bottle surface. A closed chamber with increased pressure also provides for a more sterile environment by preventing the entry of contaminating particles. The temperature differential can also be created by lowering the bottle temperature immediately prior to the introduction of the sterilizing agent.

In accordance with another aspect of the invention, an automated sterilization system is provided that can perform the foregoing sterilization methods, thereby eliminating the need for manual application of the sterilizing agent. Such a system is reliable and uses a far lower volume of sterilizing solution than prior systems.

### BRIEF DESCRIPTION OF THE DRAWINGS

FIG. 1 is a schematic fragmentary view of a portion of a sterilizing device in accordance with the present invention;
FIG. 2 is an enlarged fragmentary sectional view illustrating the initial injection of sterilizing agent into a bottle;
FIG. 3 is an enlarged fragmentary sectional view illustrating continued injection of sterilizing agent into a bottle;
FIG. 4 is an enlarged fragmentary sectional view showing a film of sterilizing agent on the bottle interior;
FIG. 5 is an enlarged fragmentary sectional view illustrating injection of a fog of sterilizing agent into the exterior of a bottle;
FIG. 6 is an enlarged fragmentary sectional view illustrating a thin film of condensed sterilizing agent on the interior bottle surface;
FIG. 7 is an enlarged fragmentary sectional view illustrating rinsing of the bottle interior;
FIG. 8 is an enlarged fragmentary sectional view illustrating gravity discharge of the rinsing fluid; and
FIG. 9 is an enlarged fragmentary sectional view illustrating removing the sterilizing agent from the bottle interior with compressed air.

### DETAILED DESCRIPTION OF THE INVENTION

The present invention provides a system, method and apparatus for sterilizing bottles by introducing a sterilizing agent in the form of discrete atomized particles onto bottle surfaces, which is particularly suited for use with a cold-fill bottling process.

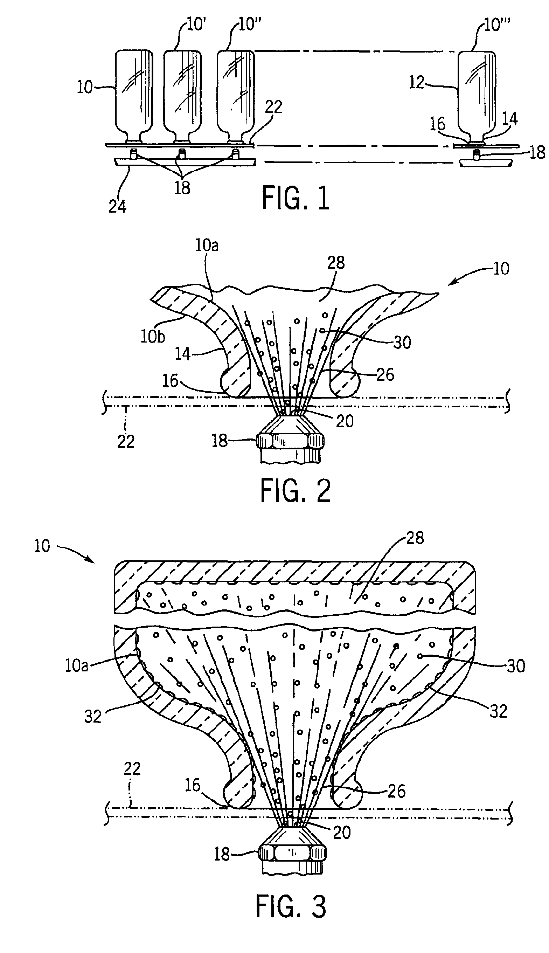

Referring to the Figures generally and in particular to Figures 1 and 2 a bottle 10 is illustrated having an interior surface 10a and an exterior surface 10b. Bottle 10 also has a body portion 12, a neck portion 14 and a mouth 16. FIG. 1 depicts bottle 10 prior to the introduction of sterilizing agent. Bottle 10 is exposed to a plurality of atomizing nozzles 18 by a conveyor 22. The plurality of nozzles 18 may be above, below, lateral to or in any combination thereof to the bottle 10. Preferably, bottle 10 is inverted before introducing the sterilizing agent. Bottles 10', 10'' and 10''' are similar to bottle 10.

Atomizing nozzles 18 are each in fluid communication with suitable piping 24 which is connected to a storage source of sterilizing agent (not shown) and is a device for producing droplets in the desired size range. The sterilizing agent may be obtained from any suitable source and stored in any suitable manner, such as in a tank, vat or other storage device, for example. Nozzles 18 each have an aperture 20 and may be any suitable type of atomizing nozzle or other device or structure known in the art for producing droplets in the desired size range. Such types of atomizing nozzles include, but are not limited to, a fog generator, an aspirator or a hydraulic atomizer nozzle. Also, ultrasonic energy can be used to form a suspension of liquid atomized particles. Any suitable method or apparatus that can atomize and deliver liquid particles in the size range of 1 millimicron to 1000 microns can be utilized in accordance with the present invention.

Preferably, atomizing nozzles 18 are air atomizing nozzles. Standard air atomizers known in the art allow the user to define a wide array of parameters. For example, the spray angle can vary from 0°, a straight-line jet, to a full-cone spray of 360°. Moreover, the desired concentration of sterilizing agent can be achieved by adjusting the sterilizing agent/air ratio of the nozzle pressure. Likewise, the flow rate and the particle size can also be varied by adjusting the nozzle discharge aperture 20.

FIG. 2 depicts nozzle 18 atomizing and beginning the injection of the sterilizing agent 26 under pressure into bottle 10. Each of nozzles 18 can be operated to continuously or intermittently discharge a stream of atomized particles of sterilizing solution so that when bottles 10-10''' traverse the conveyor and pass over nozzles 18, droplets of sterilizing agent are injected therein. Within the given size range, some particles of the atomized sterilizing agent 26 may be invisible to the naked eye while others may be visible. As such, the physical form of the atomized sterilizing agent 26 can be, but is not limited to, a fog, a vapor, a mist or an aerosol suspension. FIG. 2 shows the introduction of atomized sterilizing agent 26 into the interior 28 of bottle 10. Nozzles 18 can be arranged or configured so that atomized sterilizing agent 26 can also be applied onto the interior bottle surfaces 10a, the exterior bottle surfaces 10b, or both the interior 10a and exterior 10b bottle surfaces.

Preferably, the sterilizing agent is a solution or liquid that is effective for removing, reducing or otherwise rendering harmless microorganisms and is capable of evaporating without leaving a residue or a substantial residue on surfaces. Examples include Vortexx™ (of Ecolab Inc. of St. Paul, Minnesota) and peroxyacetic acid compositions. A preferred sterilizing agent is Oxonia™. Oxonia™, a trademark of Ecolab Inc. of St. Paul Minnesota, currently is believed to comprise an aqueous mixture of 27.5% hydrogen peroxide, 66.7% inert ingredients and 5.8% peracetic acid. The sterilizing agent may either be applied directly or mixed with water to assure the desired degree of sterility.

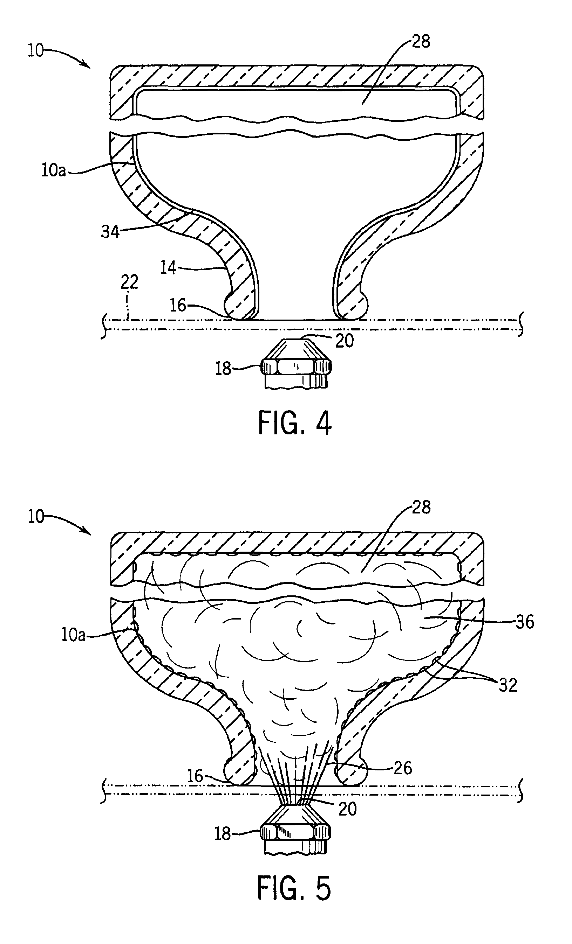

FIG. 3 depicts atomized sterilizing agent 26 being further introduced into bottle interior 28 in the form of discrete atomized particles 30. The atomized sterilizing agent particles 30 impact or otherwise contact the interior bottle surface 10a so that droplets 32 of sterilizing agent 26 form upon the interior bottle surface 10a, as shown in FIG. 3. As additional atomized particles 30 contact the interior bottle surface 10a, the droplets 32 form a continuous thin film 34 of sterilizing agent 26 upon the interior bottle surface 10a coating substantially all of the surface desired to be treated with sterilizing agent, as shown in FIG. 4. Typically, the amount of droplets to impinge upon the bottle surface will be in sufficient quantity to form a continuous thin film.

Preferably, atomized particles 30 contact the bottle's interior surface 10a by impinging thereupon. Upon impingement with interior bottle surface 10a, atomized particles 30 collapse and subsequently disperse or spread over interior bottle surface 10a. In this manner, the interior bottle surface 10a is wetted by forming a continuous thin film 34 of sterilizing agent 26 thereon. Thin continuous film 34 of sterilizing agent 26 is then allowed to remain on the interior bottle surface 10a for sufficient duration to ensure the desired degree of sterility and/or reduction of microbial contamination.

In a preferred embodiment of the present invention, the atomized sterilizing agent is introduced so as to cause it to condense upon the interior and/or exterior of desired bottle surface 10. This can be accomplished in a number of ways. First, the sterilizing agent may be introduced to the bottle surface as a supersaturated fog so that upon contact with interior bottle surface 10a, the atomized sterilizing agent 26 readily condenses thereon. Alternatively, a temperature differential between the sterilizing agent solution and the bottle temperature is created, preferably in a closed chamber with the temperature of the bottle being lower than that of the fog or droplets to promote condensation on the bottle surface. The temperature of the sterilizing agent is elevated before the sterilizing agent is atomized. Preferably the temperature of the sterilizing agent is between 16° - 82° Celcius immediately prior to atomization or as otherwise desired. The atmospheric temperature and pressure within the closed chamber can also be adjusted to further promote condensation of atomized sterilizing agent 26 upon the interior bottle surface 10a or other bottle surface.

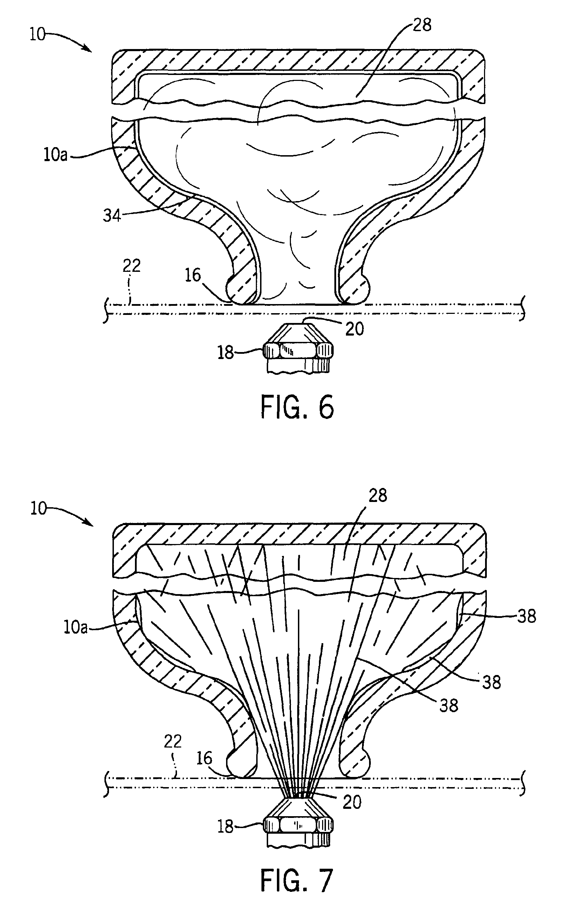

FIG. 5 depicts the atomized sterilizing agent 26 condensing as a fog 36. As quantity and volume of the atomized sterilizing agent 26 is increased, the concentration of fog 36 within the bottle interior 28 increases, reaching the atmospheric saturation point within the bottle interior 28. Once this occurs, sterilizing agent droplets 32 consequently form upon the interior bottle surface 10a. As condensation continues, droplets 32 combine to form a thin film of sterilizing agent 34 upon the interior bottle surface 10a as depicted in FIG. 6.

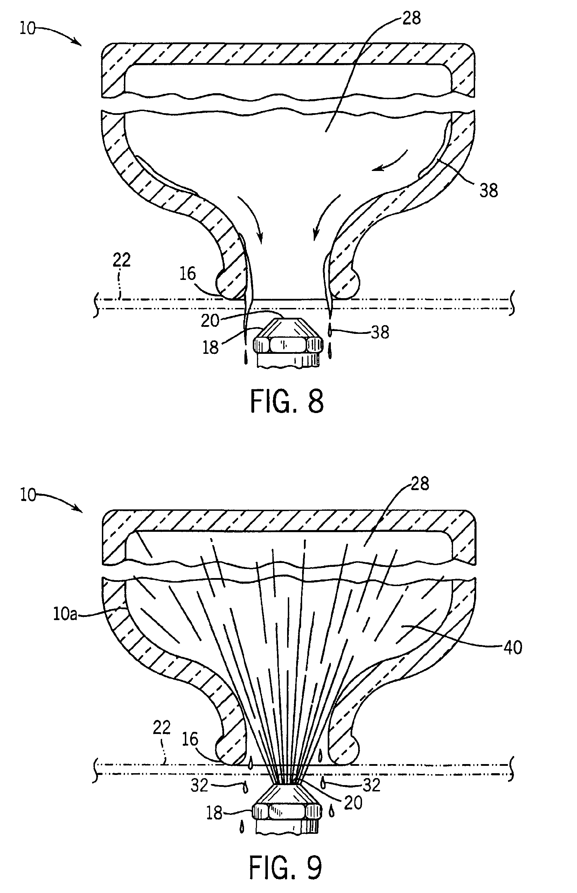

After sufficient contact time, typically on the order of about 3 seconds to about 30 seconds, for example, the film 34 of sterilizing agent is removed from the interior bottle surface 10a. The sterilizing agent may be removed in any suitable manner such as by rinsing the bottle 10 with water. FIG. 7 depicts the interior of bottle 28 being sprayed with a stream of water 38 under pressure. Preferably, the bottle is in the inverted position during rinsing thereby enabling gravity to assist the free discharge and flow of rinse water 38 through the bottle mouth 16 and off of interior bottle surfaces 10a as shown in FIG. 8.

FIG. 9 depicts one preferred embodiment wherein Oxonia™ or a similar sterilizing agent that does not leave a residue on surfaces is utilized. The sterilizing agent is removed by applying a blast of compressed air 40 onto the surfaces of bottle 10 treated with sterilizing agent for a sufficient duration and pressure to cause removal of the sterilizing agent from the bottle's interior surfaces 10a. Compressed air 40 is applied to interior bottle surface 10a to substantially evaporate any or physically remove remaining sterilizing agent. Evaporation of Oxonia™, or similar sterilizing agents, does not leave a residue.

In accordance with another aspect of the invention, the process of introducing atomized sterilizing agent 26, allowing the thin film 34 of sterilizing agent 26 to contact the interior bottle surface 10a, and removing the sterilizing agent film 34 from the interior bottle surface 10a can be automated by use of a microprocessor, computer (not shown) or the like. An automated system would not only eliminate the need for manual application of the sterilizing agent but would also produce repeatable and reliable results and can be operated on a continuous, in-line basis as part of a cold-fill bottling operation utilizing known equipment for such cold-fill bottling.

While the invention has been described with respect to certain preferred embodiments, as will be appreciated by those skilled in the art, it is to be understood that the invention is capable of numerous changes, modifications and rearrangements and such changes, modifications and rearrangements are intended to be covered by the following claims.

## Claims

1. A system for sterilizing bottles, said bottles having an interior and exterior surface, comprising:
a source of a liquid sterilizing agent;
means for introducing said sterilizing agent onto the surface of said bottle in the form of discrete atomized liquid particles by contacting the bottle surface with said particles to form at least a thin liquid film thereon, present in sufficient concentration to substantially eliminate microbial contamination on the surface of said bottle after being in contact with said liquid film for a sufficient period of time; and
means for substantially removing said sterilizing agent from said bottle surface after said bottle is sufficiently sterile.

2. The system of claim 1 or 2 wherein said contact occurs by said particles impinging and dissipating upon the bottle surface thereby substantially wetting said surface.

3. The system of claim 1 or 2 wherein said sterilizing agent is introduced so as to promote condensation of said particles onto the bottle surface.

4. The system of any previous claim wherein said sterilizing agent is introduced in a supersaturated solution to promote condensation of said particles onto the bottle surface.

5. The system of any previous claim wherein said sterilizing agent source is selected from a group consisting of an ultrasonic energy atomizer, a fog generator, hydraulic atomizer nozzle, or an air atomizer.

6. The system of any previous claim wherein said liquid droplets are in a form selected from the group consisting of a fog, a vapor, a mist, and an aerosol suspension.

7. The system of any previous claim wherein all the bottle's surfaces are contacted with sterilizing agent.

8. The system of any previous claim wherein said sterilizing agent is introduced in a closed chamber.

9. The system of claim 8 wherein said closed chamber is adapted for increased temperature and pressure to promote condensation of sterilizing agent on the surfaces of said bottle.

10. The system of claim 8 or 9 wherein the temperature of the sterilizing agent is between 16°C and 82°C.

11. The system of any previous claim wherein said sterilizing agent comprises hydrogen peroxide and peracetic acid.

12. The system of any previous claim further comprising means for inverting the bottle before introducing said sterilizing agent.

13. The system of any previous claim wherein said sterilizing agent is removed from the bottle surface by rinsing said bottle with water.

14. The system of any previous claim wherein said sterilizing agent is removed from the bottle surface with compressed air.

15. The system of any previous claim wherein said system is operated in a cold-fill liquid product filling operation.

16. A method for sterilizing bottles, said bottles having interior and exterior surfaces, comprising:
introducing a sterilizing agent in the form of discrete atomized liquid particles onto the interior bottle surface;
contacting the interior bottle surface with said particles whereby said particles form a thin liquid film on the bottle surface;
maintaining the sterilizing agent on the surface of said bottle for a fixed period of time sufficient to reduce to a desired level the amount of active microorganisms on said surface;
removing said sterilizing agent from substantially all the interior and exterior surfaces so that said surfaces are sufficiently sterile.

17. The method of claim 16 wherein said particles impinge and dissipate upon said bottle surface.

18. The method of claim 16 or 17 wherein said sterilizing agent is introduced so as to promote condensation of said particles onto the interior bottle surface.

19. The method of claim 18 wherein said sterilizing agent is introduced in a supersaturated solution to promote condensation of said particles onto the interior bottle surface.

20. The method of any of claims 16 to 19 wherein sterilizing agent is introduced by an ultrasonic energy atomizer, a fog generator, a hydraulic atomizer nozzle, or an air atomizer.

21. The method of any of claims 16 to 20 wherein said liquid particles are introduced in a form selected from the group consisting of a fog, a vapor, a mist or an aerosol suspension.

22. The method of any of claims 16 to 21 wherein said sterilizing agent is introduced in a closed chamber.

23. The method of claim 22 wherein said closed chamber is adapted for elevated temperature and pressure to promote condensation of the sterilizing agent.

24. The method of any of claims 16 to 23 wherein the sterilizing agent comprises an aqueous solution containing about 27.5% hydrogen peroxide and about 5.8% peracetic acid.

25. The method of any of claims 16 to 24 wherein said sterilizing agent is removed from the bottle surface by rinsing with water.

26. The method of any of claims 16 to 24 wherein said sterilizing agent is removed from the bottle surface with compressed air.

27. The method of any of claims 16 to 26 further comprising inverting the bottles before the introduction of said sterilizing agent.

28. The method of any of claims 16 to 27 wherein the method is operated in a cold-filling liquid product filling operation.

29. An apparatus for sterilizing bottles, said bottles having interior and exterior surfaces, comprising:
a source of a liquid sterilizing agent in the form of atomized liquid particles;
at least one nozzle for introducing said sterilizing agent onto the surface of the bottle in the form of discrete atomized liquid particles by contacting the bottle surface with said particles to form at least a thin liquid film thereon, present in sufficient concentration to substantially eliminate microbial contamination on the surfaces of said bottle in contact with said liquid film; and
a rinsing device for substantially removing said sterilizing agent from said bottle's surfaces after said bottle is sufficiently sterile.

30. The apparatus of claim 29 wherein said contact occurs by impinging and dispersing over said bottle surface.

31. The apparatus of claim 29 or 30 wherein said sterilizing agent is introduced so as to promote condensation of said particles onto the bottle surface.

32. The apparatus of claim 31 wherein said sterilizing agent is introduced in a supersaturated solution to promote condensation of said particles onto the bottle surface.

33. The apparatus of any of claims 29 to 32 wherein said source of liquid sterilizing agent comprises a device selected from the group consisting of an ultrasonic energy atomizer, a fog generator, a hydraulic atomizer nozzle and an air atomizer.

34. The apparatus of any of claims 29 to 33 wherein said atomized sterilizing agent is selected from the group consisting of a fog, a vapor, a mist and an aerosol suspension.

35. The apparatus of any of claims 29 to 34 wherein substantially all the bottle surfaces are contacted with sterilizing agent.

36. The apparatus of any of claims 29 to 35 wherein said sterilizing agent is introduced in a closed chamber.

37. The apparatus of claim 36 wherein said closed chamber is adapted for increased temperature and pressure to promote condensation of sterilizing agent on the surfaces of said bottle.

38. The apparatus of any of claims 29 to 37 wherein the temperature of the sterilizing agent is between 16°C and 82°C.

39. The apparatus of any of claims 29 to 38 wherein said sterilizing agent comprises hydrogen peroxide and peracetic acid.

40. The apparatus of any of claims 29 to 39 further comprising means for inverting the bottle before introducing said sterilizing agent.
